# FASCICULE DE BREVET EUROPEEN

(11) **EP 4 531 655 B1**
(45) Date de publication et mention de la délivrance du brevet: **13.05.2026**
(21) Numéro de dépôt: 23732212.8
(22) Date de dépôt: 30.05.2023
(51) Int. Cl.: A61B 5/00, A61B 5/12

(54) **SYSTÈME DE FIXATION À LA TÊTE D'UN UTILISATEUR DE TRANSDUCTEURS AUDIOMÉTRIQUES ET SON SYSTÈME DE CALIBRATION ADAPTABLE**
SYSTEM ZUR BEFESTIGUNG AUDIOMETRISCHER WANDLER AM KOPF EINES BENUTZERS UND KONFIGURIERBARES KALIBRIERUNGSSYSTEM DAFÜR
SYSTEM FOR FASTENING, TO THE HEAD OF A USER, AUDIOMETRIC TRANSDUCERS AND THE CONFIGURABLE CALIBRATION SYSTEM THEREOF

(30) Priorité: 01.06.2022 FR 2205290
(43) Date de publication de la demande: 09.04.2025
(73) Titulaire: My Medical Assistant, 51100 Reims (FR)
(72) Inventeur: WALLAERT, Nicolas Paul Patrice, 51100 Reims (FR)
(74) Mandataire: Radzimski, Eric
(86) Numéro de dépôt international: PCT/IB2023/055503
(87) Numéro de publication internationale: WO 2023/233275

(56) Documents cités:
- WO-A1-2008/139404
- JP-A- S56 139 740
- US-A1- 2015 358 745

## Description

La présente invention s'exerce dans le domaine des systèmes de stimulation auditive utilisables en audiométrie pour la réalisation de tests auditifs. Plus précisément, elle a d'abord trait à un procédé de calibration d'un dispositif à transducteurs audiométriques pour stimulation aérienne et stimulation vibratoire attaché à la tête de l'utilisateur pour la réalisation d'une audiométrie, et ensuite à un système de maintien sur la tête des différents dispositifs, vibrateurs et écouteurs, utilisés en audiométrie clinique pour tester bilatéralement l'audition d'un patient en stimulation aérienne et en stimulation vibratoire, sans déplacement du casque durant l'examen. En d'autres termes encore, elle concerne un système de fixation sur la tête de l'utilisateur de transducteurs pour la réalisation d'une audiométrie en conduction aérienne et en conduction osseuse, ainsi qu'un procédé de calibration du ou des transducteurs en stimulation vibratoire pour permettre leur usage lors de l'utilisation avec un tel système de fixation. Enfin, le système de fixation permet de supprimer de façon certaine la contribution d'une stimulation aérienne émanant du vibrateur survenant dans les hautes fréquences lorsqu'on ne souhaiterait tester que la conduction vibratoire.

Un audiogramme tonal, test auditif par excellence, comporte deux types d'audiométries qui permettent d'évaluer avec un degré de précision suffisant l'état de santé du système auditif d'une personne : une audiométrie en stimulation aérienne, qui teste l'intégralité des structures de l'oreille (oreille externe, moyenne et interne), et une audiométrie en stimulation vibratoire, qui teste directement l'oreille interne au moyen de vibrations. La réalisation de ces deux examens est primordiale pour déterminer le type de surdité (perte neurosensorielle, perte de transmission, perte mixte) du patient.

En général, un audiogramme de ce type est réalisé manuellement par un opérateur au moyen d'un audiomètre auquel est connecté un casque binaural en stimulation aérienne et un casque monaural en stimulation vibratoire utilisant exclusivement la conduction osseuse (placé sur le front ou l'os de la mastoïde). Un masquage controlatéral de l'oreille non testée doit par ailleurs être réalisé dans certains cas, pour s'affranchir des possibilités de réponses erronées par exemple en cas de différence interaurale conséquente, ou encore en cas de stimulation vibratoire pour laquelle le transfert transcrânien est quasiment nul. Pour la réalisation des tests, l'opérateur place sur le crâne du patient soit successivement chacun des casques lorsqu'un masquage n'est pas nécessaire, soit les deux casques concomitamment lorsqu'un masquage de l'oreille controlatérale est nécessaire pour le test en stimulation vibratoire. Dans la présente invention, comme on le verra plus en détail dans la suite, il est prévu un système d'attache et de fixation confortable, qui permet de positionner correctement et facilement sur la tête l'intégralité des transducteurs nécessaires à la réalisation d'une audiométrie binaurale en stimulation aérienne et/ou vibratoire sans repositionnement des transducteurs et sans intervention humaine.

Les demandes de brevet WO 2008/139404 A1 (KOEKEMOER, 2008-11-20), US 2015/358745 A1 (RIX ET AL, 2015-12-10) et JP S56 139740 A (YAMASHITA ET AL, 1981-10-31) sont pertinentes pour comprendre le cadre de l'invention.

Le choix du positionnement du vibrateur du transducteur en stimulation vibratoire et/ ou le type de casque utilisé en stimulation aérienne imposent une exigence particulière quant à la calibration des transducteurs, en raison d'une modification de l'occlusion de l'oreille induite par la cohabitation entre les différents transducteurs puisqu'il y a, contrairement aux solutions existantes, port simultané des transducteurs de la stimulation aérienne et vibratoire sur l'oreille testée.

L'invention concerne par conséquent un procédé de calibration d'un dispositif à transducteurs audiométriques pour stimulation aérienne et stimulation vibratoire attaché à la tête de l'utilisateur pour la réalisation d'une audiométrie par émission de signaux à des fréquences inférieures à 20 kHz en direction de la tête, comportant au moins un transducteur vibratoire de type vibrateur en conduction osseuse et un appareil de stimulation aérienne muni d'au moins un transducteur, dans un casque auditif ou sous forme d'un insert auditif, le procédé de calibration consistant en :
- la mise en place du ou des vibrateurs en conduction osseuse sur la tête du patient ;
- la mise en place du casque / des inserts en conduction aérienne sur le patient ;
- la sélection des paramètres suivants :
   ● un ou plusieurs tests à réaliser parmi les tests en stimulation aérienne ou en stimulation vibratoire et, pour chacun desdits tests,
   **●** le ou les oreilles à tester,
   **●** la présence ou non d'un masquage controlatéral ;
- la sélection du type de transducteur utilisé et de leur positionnement ;
- la modification de la calibration des transducteurs selon le transducteur et les paramètres sélectionnés, par application d'une pondération fréquentielle globale des signaux ;
- la génération par les transducteurs des signaux fréquentiels, constituant les stimuli de la stimulation vibratoire et/ou aérienne.

Le principe d'une pondération fréquentielle, c'est que l'on applique sur une valeur donnée une modification de la valeur de l'intensité du son qui dépend de la fréquence. Le bruit se mesure en dB, 0 dB représentant le seuil d'audibilité. Par exemple, si la mesure donne une valeur non pondérée de dB pour différentes fréquences (e.g. 1000, 2000, 4000Hz...), avec une pondération fréquentielle de 10dB à 1000Hz, 20dB à 2000Hz, et 30dB à 4000Hz, le niveau d'intensité APRES pondération sera donc de 10+10=20dB à 1000Hz ; 10+20 = 30dB à 2000Hz et 10+30 = 40dB à 4000Hz.

L'objectif de la pondération est d'adapter le niveau d'intensité du signal sonore en fonction de la condition dans laquelle l'examen est réellement réalisé, qui diffère de la norme (par exemple du fait de la création d'un effet d'occlusion, ou autre : voir les exemples ci-après). La pondération globale dépend donc du type de transducteurs, de leur positionnement, de leur couplage, etc.

Il est par ailleurs précisé qu'on entend par pondération globale un cumul de différentes pondérations qui peuvent s'appliquer selon le contexte du test : pour une situation donnée, il est parfois nécessaire de sommer les coefficients de pondération de façon à prendre en compte la situation testée (voir les explications détaillées ci-après). La somme des différentes pondérations est appliquée sur la valeur de référence, fournie par la norme qui ne prévoit qu'une condition expérimentale de mesure (0dB de référence, dépendant du type de transducteur utilisé pour un positionnement donné, oreille testée non occluse).

Le côté de la tête à tester en premier pour un test en stimulation vibratoire résulte de la réalisation d'un test de Weber incluant une stimulation en conduction osseuse bilatérale ou frontale à une intensité supraliminaire à différentes fréquences audiométriques, par exemple 0,5kHz, 1kHz, 2kHz, 4kHz, permettant de déterminer un premier côté de test selon le côté de perception par l'utilisateur de la stimulation. Il s'agit d'une détermination connue en soi.

En général, le transducteur vibratoire est placé sur au moins un emplacement de la tête sélectionné parmi les suivants :
- le front ;
- la mastoïde ;
- une zone couvrant une portion cartilagineuse de la tête.

Ainsi, plus précisément :
- lorsque la stimulation par transducteur vibratoire a lieu sur le front, la pondération fréquentielle appliquée est comprise entre [-10 et +20] dB ;
- lorsque la stimulation par transducteur vibratoire a lieu sur un mastoïde, la pondération fréquentielle appliquée est comprise entre [-20 et +10] dB ;
- lorsque la stimulation par transducteur vibratoire a lieu sur une zone couvrant une portion cartilagineuse de la tête, la pondération fréquentielle appliquée est comprise entre [-40 ; 0] dB.

La pondération fréquentielle additionnelle appliquée est quasi nulle, c'est-à-dire comprise entre [-5 ; 5] dB si un masquage controlatéral est effectué via l'appareil de stimulation aérienne placé sur l'oreille controlatérale uniquement, l'oreille testée restant libre.

Pour mémoire, un masquage controlatéral s'obtient en présentant un message acoustique à une oreille pour éviter qu'elle puisse répondre à la place de l'oreille testée.

La pondération fréquentielle appliquée est comprise entre [-30 ; +10] dB si un masquage controlatéral est effectué via l'appareil de stimulation aérienne placé simultanément sur les deux oreilles et que le transducteur vibratoire est positionné sur le front, sur la (les) mastoïde(s) ou sur une zone couvrant une portion cartilagineuse de la tête.

Il est précisé que les pondérations précédentes peuvent être cumulatives, c'est-à-dire que l'on peut, pour une situation donnée, sommer les coefficients de pondération de façon à prendre en compte la situation testée. Par exemple, si l'examen en conduction osseuse est réalisé en plaçant le vibrateur en position frontale (pondération A), en présence d'un masquage controlatéral réalisé en positionnant le casque aérien sur les deux oreilles simultanément (pondération B), la pondération globale sera la somme fréquence par fréquence de chacune des pondérations individuelles (somme fréquence par fréquence de la pondération A et de la pondération B et de la pondération C).

Le coefficient de pondération global précédemment obtenu vise à annuler les variations de calibration et de zéro de référence induites par un positionnement différent du ou des vibrateurs osseux et transducteurs aériens de celui prescrit dans la norme.

Le niveau d'intensité requis pour des conditions expérimentales données lors d'un test auditif utilisant la conduction aérienne et la conduction osseuse peut-être :
- Soit sauvegardé en mémoire tel quel en incluant une table de calibration pour chacune des conditions expérimentales, dont les cibles à atteindre sont déterminées en appliquant la norme ISO 389-3:2016, auxquelles on applique la pondération globale précédemment calculée (calibrations mères multiples). On dénomme calibration mère la création d'une table de calibration de taille n, qui contient toutes les combinaisons expérimentales possibles (n = occlusion x positionnement x masquage x...). En somme, pour chacune des conditions, on enregistre une valeur que l'on calibre. En d'autres termes encore, cela revient à créer autant de tables de calibration qu'il pourrait y avoir de conditions expérimentales, les valeurs cibles pour chacune des tables correspondants alors à la valeur ISO 389-3:2016 à laquelle est appliquée la pondération globale.
- Soit calculé préalablement à la génération du stimulus, en appelant le niveau d'intensité sauvegardé dans la calibration (norme ISO 389-3:2016, calibration mère unique), à laquelle on vient appliquer la pondération globale (calibrations filles multiples). On entend par calibration fille le fait de réaliser une seule fois la calibration (ISO), puis on calcule à partir de cette calibration unique le niveau de sortie désirée en lui appliquant la pondération.

Dans les deux cas de figure, le résultat obtenu est rigoureusement identique (la pondération globale est appliquée soit lors de la phase de calibration du matériel, soit lors de la phase de génération du stimulus).

En pratique, différentes solutions de mise en application sont possibles et proposées à titre d'exemples non limitatifs. Elles peuvent être utilisées séparément ou de façon combinée, pour garantir une fiabilité des seuils audiométriques obtenus lors des mesures.

Ainsi, le niveau de stimulation final peut être appliqué :
Soit en modifiant le niveau d'intensité du signal émis, c'est-à-dire de la stimulation cible osseuse fournie par la norme ISO 389-3:2016, et en lui appliquant la pondération globale (calibration fille) ou en appelant directement la calibration correspondante dans la table (calibration mère). Par exemple, si la norme ISO stipule qu'un niveau de 78,9 dB SPL à 500Hz correspond à 40 dB HL et que la pondération globale issue de la configuration de test est de -16 dB à cette fréquence, alors l'intensité de stimulation sera de 78,9 -16= 62,9 dB SPL. La valeur affichée sur l'interface utilisateur reste 40dB HL. Le niveau de présentation du signal cible est abaissé.

Soit encore en modifiant le niveau d'intensité de la stimulation masquante aérienne produite dans l'oreille controlatérale, de façon à empêcher la détection du signal cible osseux par l'oreille controlatérale. Pour rappel, l'intensité du masquage controlatéral doit être comprise entre le critère d'efficacité (intensité de masquage minimale nécessaire pour empêcher la détection du son cible par l'oreille controlatérale) et le critère de retentissement (intensité de masquage maximale au-delà de laquelle le son masquant présenté dans l'oreille controlatérale commence à retentir sur la détection du son cible, présenté dans l'oreille testée). Dans la présente invention, le niveau d'intensité de la stimulation masquante peut être modifié en fixant le niveau du bruit masquant au seuil de retentissement (pourvu que le critère de retentissement soit supérieur au critère d'efficacité, auquel on soustrait le coefficient de pondération global. Par exemple, si le seuil de retentissement fixe le niveau d'intensité du bruit masquant à 60dB HL, et que la pondération globale est de -16dB à la fréquence testée (ce qui signifie que le seuil de détection de la conduction osseuse dans les conditions de tests s'améliore de 16dB comparativement à la norme), le bruit de masquage sera alors de 60 - ( -16) = 76dB HL. En retentissant, le niveau de bruit masquant généré contraindra la détectabilité du son cible présenté dans l'oreille testée et modifiera alors le seuil obtenu d'une valeur égale à la pondération globale.

Soit enfin en modifiant la valeur affichée une fois le résultat de la mesure effectuée en présentant un niveau d'intensité de stimulation pour le son cible et pour le son masquant en accord avec la norme ISO 389-3:2016. La réponse du patient est simplement enregistrée en prenant la valeur du son cible et en lui ajoutant la pondération globale. Par exemple, si le patient répond à 40dB HL et que la pondération globale pour la condition expérimentale testée à la fréquence testée est de -16dB, alors la réponse du patient sera enregistrée à 40-16 = 24 dB HL.

Il est possible d'utiliser toutes combinaisons d'au moins deux des trois types d'application pour arriver au même résultat. Par exemple, si le facteur de pondération est de -16 dB, il est possible d'appliquer -8 dB au niveau d'intensité du son cible et +8 dB au niveau d'intensité du son masquant.

Pour mémoire, on rappelle que :
Les dB HL sont nommées d'après l'acronyme anglo-saxon « Decibel Hearing Level », et les dB SPL sont désignés d'après l'acronyme « Decibel Sound Pressure Level ». En fait, un niveau de pression sonore s'exprime en décibels physiques (dB SPL). Zéro dB SPL est le seuil d'audition de référence à 1kHz, donc obtenu sur un groupe de sujets normaux. C'est dire que l'échelle des décibels acoustiques n'existerait pas sans l'oreille humaine. Ce zéro correspond à une pression de 20µPa, l'unité de pression étant le Pa (Pascal). Zéro Pa ne peut exister que dans le vide et cette unité ne peut donc pas être utilisée comme zéro audiométrique. Comme l'oreille n'est pas également sensible à toutes les fréquences, il a fallu exprimer les zéros audiométriques (les seuils d'audition pour chaque fréquence) en dB SPL. En audiométrie tonale, la mesure des seuils d'audition, c'est-à-dire l'audiométrie liminaire, se fait donc par référence aux zéros audiométriques. Les résultats de mesures s'expriment alors en dB HL.

Comme évoqué, les facteurs correctifs ou les nouvelles calibrations sont dépendantes du couplage acoustique des transducteurs, et peuvent être amenées à varier en fonction du type de transducteur utilisé et de leur positionnement, impactant l'effet d'occlusion crée par la cohabitation des différents transducteurs, permise par le système de fixation de l'invention.

L'introduction d'une calibration multiple des transducteurs en conduction osseuse en fonction du type de transducteurs aériens utilisés doit être implémentée de façon à normaliser les seuils liminaires obtenus dans les différentes conditions expérimentales qui pourront être rencontrées (casque binaural en stimulation vibratoire porté seul, ou alternativement avec adjonction du casque binaural en stimulation aérienne, que ce soit casque ou insert auriculaire).

A noter que dans les hautes fréquences, l'effet d'occlusion peut être négatif, c'est-à-dire que l'occlusion du conduit auditif externe dégrade la perception « vibratoire » des aigus. En réalité, l'occlusion du conduit dégrade la perception du champs acoustique rayonné par le vibrateur, qui s'avère audible par le sujet (stimulation aérienne émise involontairement par le vibrateur). Le système de support qui va être décrit dans la suite, avec la cohabitation entre dispositif de stimulation aérienne et vibratoire, permet d'empêcher le sujet de percevoir le rayonnement aérien émis par le vibrateur. La qualité du diagnostic en est améliorée, les seuils mesurés en stimulation vibratoire n'étant alors plus que le reflet de la réponse de l'oreille interne, y compris dans les hautes fréquences.

Actuellement, les transducteurs en stimulation vibratoire utilisables en audiométrie présentent tous le même système d'attache, à savoir un serre-tête sur lequel est fixé un seul transducteur. Aucun système d'audiométrie binaural en stimulation vibratoire n'est en effet disponible à l'heure actuelle, probablement en raison de différents problèmes techniques déjà évoqués ci-dessus et qui connaissent bien entendu une traduction au niveau des dispositifs placés sur la tête des patients, dont :
i) Les systèmes actuels d'audiométrie ne proposent qu'une calibration unique pour chaque transducteur, que cela soit en conduction aérienne ou vibratoire. Les références issues de la norme ISO 389-3:2016 permettent de calibrer le vibrateur pour une oreille ouverte (non occluse par un casque). La création d'une occlusion induite par la cohabitation entre les deux casques aérien et vibratoire, même partielle, modifie sensiblement le niveau sonore perçu par le patient (et donc les 0 dB de référence utilisé), anéantissant alors le bénéfice de la calibration.
ii) Le serre-tête est calibré pour fournir une pression « suffisante » pour les besoins de l'audiométrie. Plus précisément, une force d'application comprise entre 4,9 à 5,9 Newtons est normalement exercée pour permettre ce type de test, force qui varie cependant en pratique d'un individu à l'autre en fonction de son périmètre crânien. Dans les matériels existants, la force de serrage du serre-tête n'est jamais ajustée et n'est pas réglable. De plus, le dispositif actuel est rapidement inconfortable au delà de quelques minutes de port, ce qui ne le rend pas compatible avec la durée plus conséquente nécessaire pour la réalisation d'un examen audiométrique complet de bout en bout.

Or, la pression de serrage des transducteurs en stimulation vibratoire est un paramètre important pour la transmission physique des signaux. Chaque transducteur comporte à cet effet une surface plane et circulaire de 150 à 200 mm² qui vient en appui sur la tête du patient, en général sur l'apophyse mastoïde. Lorsque l'intégralité de la force de serrage résultant des casques monauraux actuels (par exemple lorsque le serre-tête est métallique rigide) est appliquée, elle provoque une forte pression sur la mastoïde, qui rend le port du casque difficilement supportable car très inconfortable, voire douloureux. La pose des casques peut donc s'avérer délicate et instable, et l'opérateur qui s'en charge doit faire preuve d'un certain savoir-faire, d'autant qu'il est nécessaire de le repositionner à plusieurs reprises de façon à tester chacune des deux oreilles pour la réalisation de l'ensemble des tests. Les tests en conduction vibratoire devant être réalisé oreille ouverte, il convient de positionner simultanément de façon croisée le vibrateur (ossivibrateur sur mastoïde et coussin d'appui sur la mandibule) et le casque aérien en diagonale (i.e. un écouteur sur l'oreille à masquer et l'autre sur la joue, pour laisser l'oreille testée ouverte).

La nécessité de laisser une oreille ouverte, le confort rudimentaire et l'obligation de changer le casque de côté en stimulation vibratoire ne permettent pas de réaliser l'intégralité des tests sans retirer le casque, imposant la présence d'un opérateur sur place pour assurer le repositionnement des transducteurs au long des différentes phases du test. Si un positionnement frontal du vibrateur, évoqué par certains auteurs (par exemple Studebaker 1962) pourrait permettre de réaliser l'examen sans intervention extérieure et modification du positionnement des casques, les résultats obtenus dans de telles circonstances pour la réalisation de l'examen en stimulation vibratoire serait erronés notamment en raison d'une calibration de l'ossivibrateur pour une stimulation oreille ouverte et non occluse ou semi occluse, sauf à décaler le casque sur l'oreille non testée pour la laisser ouverte.

Ces différentes problématiques n'ont jusqu'à présent jamais été résolues, notamment en raison d'une absence de besoin, les examens étant réalisés en présentiel et le positionnement des casques pouvant être modifiés par l'expérimentateur sans difficultés. Or, de nos jours, l'avènement de l'intelligence artificielle et de la télémédecine permettrait d'automatiser une partie des tests audiométriques et de les réaliser éventuellement à distance. Au moins une partie des bénéfices que l'on pourrait attendre de l'automatisation et de la télémédecine (gain de temps humain) ne sont donc pas envisageables à cause des contraintes mentionnées ci-dessus, tout particulièrement du fait de la nécessité de la présence d'une personne s'occupant des opérations de pose et repose du casque sur le crâne du patient, ou de l'impact acoustique majeur d'une modification du positionnement des vibrateurs.

La présente invention se propose de remédier aux inconvénients précités en proposant un système d'attache et de fixation confortable qui permet de positionner correctement et facilement sur la tête l'intégralité des transducteurs nécessaires à la réalisation d'une audiométrie binaurale en stimulation aérienne et/ou vibratoire sans repositionnement des transducteurs et sans intervention humaine (sans déplacement du système pendant le test) au cours de l'examen. Pour répondre à cet objectif, plus particulièrement, l'invention présente également un système de maintien des transducteurs plus sûr, car mieux adapté à la forme individuelle de la tête et des oreilles. Il en résulte un confort amélioré, permettant au destinataire de garder ladite invention durant l'intégralité des examens sans aucun déplacement. Le système proposé fonctionne en particulier avec le procédé de l'invention, qui est en réalité un procédé d'ajustement de la calibration initiale de l'ossivibrateur, laquelle permet de compenser les variations liées aux différences entre les conditions de mesures réelles et les conditions issues de la norme fixant le 0dB de référence (occlusion du conduit auditif externe en pratique versus conduit auditif ouvert dans la norme).

En fait, dit autrement, il faut pouvoir tester les deux oreilles binauralement et simultanément en stimulation aérienne et/ou vibratoire, avec ou sans masquage controlatéral, après installation simultanée ou quasi simultanée des transducteurs nécessaires (c'est-à-dire d'au moins un transducteur pour la stimulation vibratoire et deux transducteurs pour la stimulation aérienne) de façon à permettre de réaliser l'intégralité de l'examen sans intervention extérieure. Dans le cas d'un unique transducteur en stimulation vibratoire, les excellentes propriétés de transmission d'une stimulation vibratoire dans l'os (transfert transcrânien très faible autour de 0 à 10dB, cf. Annexe 1) ou dans les tissus mous sont ici utilisées pour transmettre le son de façon bilatérale.

Dans le cas d'un seul transducteur pour la stimulation vibratoire, le vibrateur pourra par exemple alors être positionné sur le crâne à équidistance des deux oreilles (pour un patient vu de face : azimut 0°, c'est-à-dire sur l'os frontal dans l'axe de suture frontale, ou 180° sur l'os occipital, dans l'alignement de la suture sagittale et sous la suture lambdoïde). Dans le cas de deux transducteurs pour la stimulation vibratoire, le positionnement des vibrateurs pourra par exemple être réalisé précisément en regard de l'apophyse mastoïdienne de part et d'autre du crâne. Quelque soit le nombre de transducteur(s) utilisé(s) pour la stimulation vibratoire, deux transducteurs en stimulation aérienne sont ajoutés au précédent dispositif, soit de façon intra-auriculaire (inserts auriculaires), soit de façon supra auriculaire (casque audio).

Pour remplir ces objectifs, et d'autres qui seront apparents à la lecture de ce texte, le système de l'invention, assurant la fixation à la tête d'un utilisateur de transducteurs pour la réalisation d'une audiométrie en stimulation aérienne et en stimulation vibratoire, est tel qu'il comporte des moyens support de deux transducteurs audiométriques en conduction aérienne chacun positionnable au niveau d'un des pavillons auditifs ou du conduit auditif externe et d'au moins un transducteur audiométrique en stimulation vibratoire.

Le système de l'invention comporte par conséquent au moins trois transducteurs permettant une stimulation binaurale tant pour la stimulation aérienne que pour la stimulation vibratoire (conduction osseuse et/ou par tissu mou). Il est donc fait pour stimuler le système auditif pour les deux types de stimulations, permettant dès lors une exploration fonctionnelle auditive donnant des informations exhaustives pour réaliser un diagnostic abouti. Il se distingue des solutions actuelles d'audiométrie en stimulation vibratoire qui permettent des stimulations osseuses monaurales dans le respect de la norme NF EN ISO 389-3 ou des stimulations binaurales, mais pour lesquelles la modification des conditions expérimentales induit une modification substantielle des niveaux de référence équivalents de force vibratoire liminaire pour les vibrateurs à sons purs lorsque l'oreille est occluse.

Les solutions actuelles d'audiométrie en stimulation vibratoire ne permettent donc pas des stimulations osseuses binaurales, sans déplacement des différents casques. Comme précisé auparavant, les moyens support des deux types de transducteurs permettent de réaliser l'intégralité de test sans qu'il soit nécessaire de les retirer, après leur installation initiale : lesdits moyens support doivent donc être conçus pour qu'une fois installés, ils garantissent le positionnement correct des transducteurs et leur maintien dans le temps. Enfin, un algorithme de compensation de calibration permet de s'affranchir de l'effet d'occlusion crée par la cohabitation des différents casques audiométriques.

Selon une possibilité, les moyens supports comportent une combinaison de premiers moyens support pour les transducteurs audiométriques en stimulation aérienne et de seconds moyens support pour le ou les transducteurs audiométriques en stimulation vibratoire. Il revient à l'utilisateur de les positionner initialement, le cas échéant l'un relativement à l'autre, sans qu'il y ait ensuite nécessité de les repositionner jusqu'à la fin des tests.

De fait, selon une configuration possible, les premiers moyens support des transducteurs audiométriques en stimulation aérienne et les seconds moyens supports du ou des transducteurs audiométriques en stimulation osseuse sont solidarisés l'un à l'autre, de sorte qu'il n'y a aucun besoin de les positionner relativement l'un à l'autre. Le casque à au moins trois transducteurs qu'ils forment est en effet manipulable en une seule pièce qu'il convient de fixer sur la tête, en ajustant les positions des transducteurs par rapport au conduit auditif ou aux pavillons des oreilles d'une part pour la conduction aérienne, et des mastoïdes, du front pour la conduction vibratoire d'autre part.

En pratique, diverses configurations peuvent être mises en œuvre dans le cadre de l'invention. Ainsi, selon un premier exemple, les seconds moyens supports pour les transducteurs audiométriques en stimulation vibratoire peuvent être constitués d'un bandeau positionnable sur le front (un vibrateur) ou en regard des apophyses mastoïdiennes en arrière des pavillons auriculaires. Ledit bandeau peut être souple ou rigide, et son positionnement ainsi que la force de serrage exercée sur le crâne peut varier, au gré de l'utilisateur, principalement selon sa morphologie crânienne et l'emplacement des pavillons. Dans le présent cas, le système d'attache des vibrateurs peut être réalisé soit au moyen d'un système de serrage, utilisant des crans prévus à cet effet de part et d'autre du vibrateur, soit simplement en utilisant la force de serrage du bandeau et en glissant l'ossivibrateur sous le bandeau.

Alternativement, lesdits seconds moyens supports pour les transducteurs audiométriques en stimulation vibratoire peuvent être constitués d'un arceau semi-rigide entourant la nuque et dont les extrémités sont munies d'embouts souples placées au-dessus des pavillons auriculaires. Bien que semi-rigide, l'arceau peut facilement être déplacé pour s'adapter au crâne du patient.

En général, selon l'invention, les premiers moyens supports pour les transducteurs audiométriques en stimulation aérienne consistent en un casque comportant un arceau supérieur muni de moyens réglables de sa longueur et soit d'écouteurs placés sur les pavillons auriculaires, soit d'inserts insérés directement dans le conduit auditif. Ledit casque est positionné en lien avec les seconds moyens supports pour les transducteurs audiométriques en stimulation vibratoire présentés ci-dessus, pour un positionnement global des différents transducteurs.

Dans certaines configurations, l'arceau du casque peut comporter, une branche se développant en direction du front ou d'un os mastoïde (stimulation osseuse) ou de la région pré-tragienne (stimulation par tissus mous) sur laquelle est solidarisé un transducteur audiométrique permettant une stimulation vibratoire. Il s'agit de la configuration en une seule pièce.

De manière générale, selon l'invention, les transducteurs sont fixés à leurs supports via des moyens de fixation dont la position est réglable par rapport auxdits supports. Il est aisé à l'utilisateur de les déplacer pour les positionner correctement, c'est-à-dire de manière adaptée à son crâne et, de chaque côté, à l'emplacement du pavillon auriculaire ou du conduit auditif externe et des zones des stimulations vibratoires (de façon non limitative l'os de la mastoïde, front, tissus mous du crâne...).

Le choix du positionnement du vibrateur du transducteur en stimulation vibratoire et/ ou le type de casque utilisé en stimulation aérienne imposent une exigence particulière quant à la calibration des transducteurs, en raison d'une modification de l'occlusion de l'oreille induite par la cohabitation entre les différents transducteurs - puisqu'il y a, contrairement aux solutions existantes, port simultané de la stimulation aérienne et vibratoire.

D'autres buts et avantages de la présente invention apparaîtront au cours de la description qui va suivre, se rapportant à des modes de réalisation qui ne sont donnés qu'à titre d'exemples indicatifs et non limitatifs.

La compréhension de cette description sera facilitée en se référant aux dessins joints en annexe et dans lesquels :
La [Fig.1] représente une vue schématisée de profil d'un utilisateur équipé de moyens supports constitués d'un bandeau pour les transducteurs audiométriques en stimulation vibratoire (conduction osseuse et par tissus mous).
La [Fig.2] représente une vue schématisée arrière de l'utilisateur équipé dudit bandeau support.
La [Fig.3] représente une vue schématisée de face de l'utilisateur équipé dudit bandeau support lorsqu'un seul vibrateur est utilisé, mais positionné de façon frontale.
La [Fig.4] représente une vue schématisée de profil d'un utilisateur équipé de moyens supports, également pour les transducteurs audiométriques en stimulation vibratoire, constitués d'un arceau semi-rigide entourant la nuque d'un utilisateur.
La [Fig.5]] représente une vue schématisée arrière de l'utilisateur équipé dudit arceau support.
La [Fig.6] représente une vue schématisée de profil d'un utilisateur équipé d'un casque combiné, doté d'un arceau supérieur pour les transducteurs audiométriques en stimulation aérienne (écouteurs) et de branches support pour les transducteurs audiométriques en stimulation vibratoire.
La [Fig.7] représente une vue schématisée arrière de l'utilisateur équipé dudit casque combiné de la figure précédente.

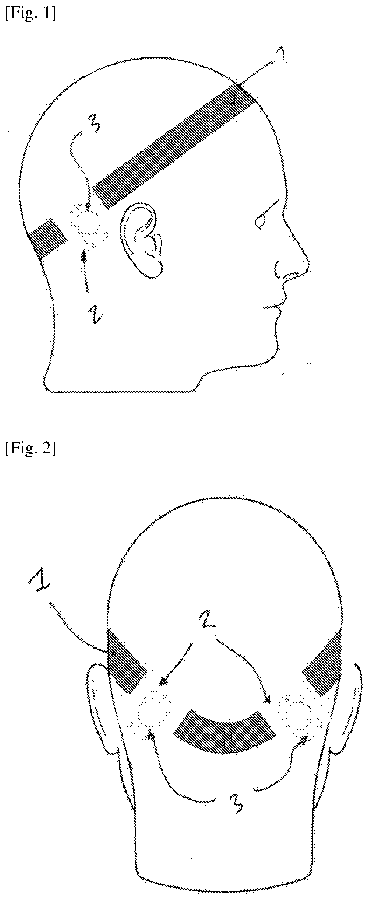

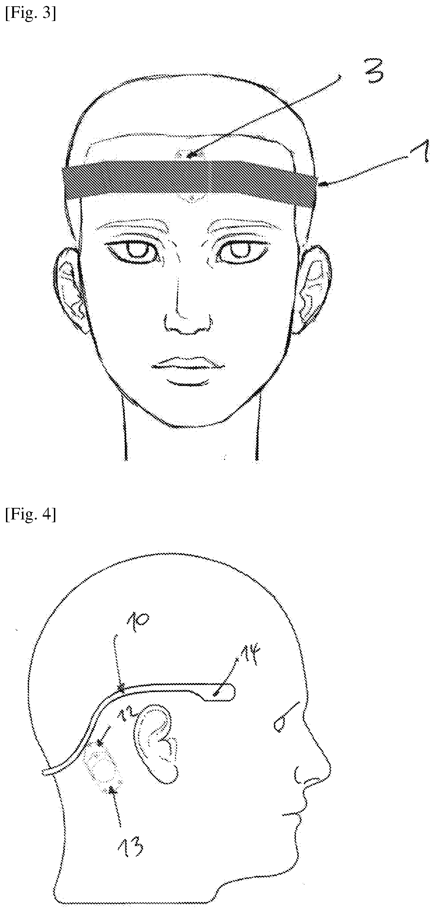

La configuration des [Fig.1] à [Fig.3] montre un bandeau souple ou rigide. Ce support particulier est conçu pour laisser dégagés les pavillons auriculaires du patient de façon à y adjoindre un casque ou des inserts en stimulation aérienne binaural permettant de compléter les tests audiométriques. Sa nature et son positionnement permettent de l'installer simultanément à un casque traditionnel muni d'écouteurs ou d'inserts. Le bandeau 1 peut selon un exemple de réalisation comporter des moyens de fixation 2 pour le ou les transducteurs 3 à simulation vibratoire qui assurent le maintien immobile de ceux-ci sur le bandeau 1. Le ou les transducteurs 3 à stimulation vibratoire peuvent également être glissés sous le bandeau, sans aucun système de fixation (voir en [Fig.3]), leur maintien étant alors assuré par la force de pression du bandeau. Ces moyens de fixation 2 peuvent être ajustés/coulissés sur le bandeau de façon à modifier, en vue d'un réglage, la distance entre le ou les transducteurs 3 afin qu'ils soient bien positionnés, en général en regard de la zone de stimulation (par exemple l'apophyse mastoïde de l'os temporal, le front ou éventuellement des tissus mous), et/ou - dans le cas d'un bandeau souple - la force de serrage exercée par le système.

A cet égard, un ou plusieurs systèmes de réglages de la force de serrage exercée peu(ven)t être mis en œuvre : ces systèmes peuvent par exemple être constitués de glissières, de bouton-pressions, de crans de réglage, etc. Ces systèmes de réglages permettent en pratique d'ajuster la pression exercée par les transducteurs à stimulation vibratoire sur l'os ou aux tissus mous du patient.

La taille du bandeau peut être universelle ou réglable et, dans le cas d'un bandeau 1 souple, la force de pression peut éventuellement également être ajustée en mesurant le périmètre crânien du patient via un outil de mesure. Le résultat de la mesure permet d'ajuster de manière plus fine le diamètre du bandeau via les systèmes de réglages et des marques positionnées sur le bandeau jusqu'à atteindre la force de pression nécessaire, celle qui correspond au périmètre crânien mesuré. Le bandeau 1 peut éventuellement comporter un système d'ouverture (coulisseau, clips, fermeture auto-grippante, ...) pour faciliter sa mise en place sur le patient.

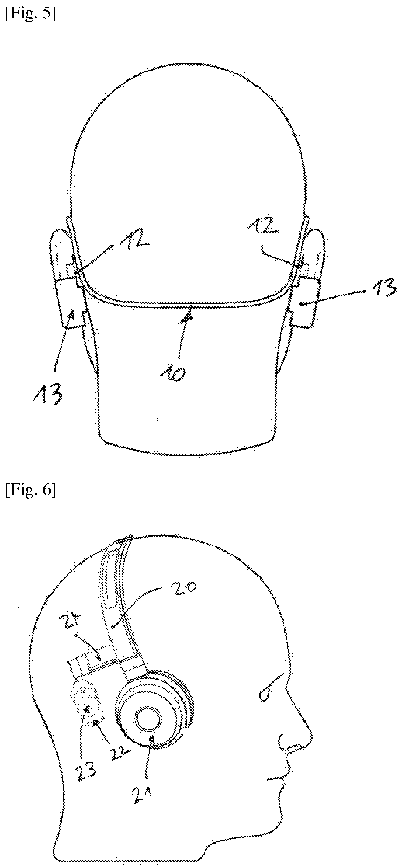

Les moyens supports illustrés en [Fig.4] et [Fig.5] comportent un arceau semi rigide 10 conçu pour être positionné en arrière de la tête du patient. Comme pour la configuration en bandeau 1 des [Fig.1] à [Fig.3], ces moyens supports sont conçus pour laisser dégagés les pavillons auriculaires du destinataire de façon à y adjoindre un casque écouteurs ou des inserts, ces derniers constituant les transducteurs en stimulation aérienne binaural. Le positionnement de l'arceau 10 arrière permet en pratique d'installer ces seconds moyens support simultanément à une disposition en stimulation aérienne traditionnel.

Le système de fixation se compose en l'espèce d'un arceau 10 semi rigide, malléable de façon à être ajustable à la morphologie du patient, auquel sont ajoutés différents éléments. Cet arceau 10 peut être proposé éventuellement en différentes tailles. L'arceau 10 doit être placé juste au-dessus des pavillons des oreilles et doit suivre le contour de la tête. Un ou des moyens de fixation 12 permet(tent) de fixer le ou les vibrateurs 13 constituant les transducteurs à stimulation vibratoire sur l'arceau 10. Ces moyens de fixation 12 sont mobiles sur l'axe de l'arceau 10 pour permettre leur positionnement en regard de l'apophyse mastoïde de l'os temporal ou éventuellement de la zone prétragienne pour la stimulation par tissus mous. Deux embouts souples 14 permettent par ailleurs le maintien de l'ensemble en position. Les embouts souples 14 doivent reposer légèrement en avant de l'oreille de façon à être confortables tout en maintenant un appui des vibrateurs 13 sur la zone de stimulation.

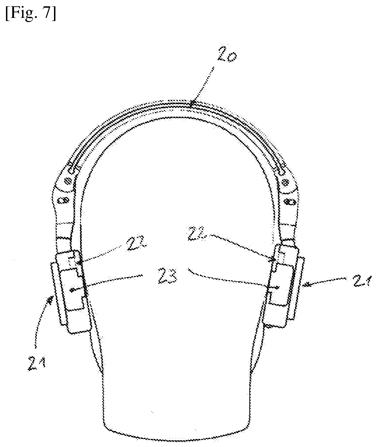

Une troisième configuration combinée apparaît en [Fig.6] et [Fig.7], dans laquelle les moyens support sont basés sur un casque muni d'écouteurs 21 de stimulation aérienne. Le dispositif de l'invention est constitué d'un arceau supérieur 20, qui constitue de fait les moyens support et peut être télescopique, c'est-à-dire comprendre des bras extensibles pour une adaptation à la taille de la tête de chaque patient. Les bras extensibles de l'arceau supérieur 20 sont terminés à chaque extrémité par un écouteur 21 professionnel pour la conduction aérienne et par un vibrateur audiométrique 23 pour la conduction osseuse. Un autre exemple de l'invention serait d'avoir un ou plusieurs autres bras extensibles de l'arceau supérieur orienté en direction des zones de stimulations choisies (par exemple front, mastoïdes, zones prétragiennes, ...). Les vibrateurs 23 constituant les transducteurs à simulation vibratoire sont placés sur des moyens de fixation 22 disposés à l'extrémité libre de branches 24 les reliant aux bras de l'arceau supérieur 20.

Dans tous les cas évoqués, les transducteurs sont destinés à être connectés à un audiomètre conventionnel par des connecteurs filaires ou par une technologie de transmission des signaux sans fil.

Les exemples ci-dessus ne sont pas limitatifs de l'invention, qui englobe au contraire les variantes de forme et de configuration qui sont dans la portée de l'invention.

## Revendications

1. Procédé de calibration d'un dispositif à transducteurs audiométriques pour stimulation aérienne et stimulation vibratoire attaché à la tête de l'utilisateur pour la réalisation d'une audiométrie par émission de signaux à des fréquences inférieures à 20 kHz en direction de la tête, comportant au moins un transducteur vibratoire de type vibrateur en conduction osseuse et un appareil de stimulation aérienne muni d'au moins un transducteur, dans un casque auditif ou sous forme d'un insert auditif, le procédé de calibration consistant en :
- la mise en place du ou des vibrateurs en conduction osseuse sur la tête du patient ;
- la mise en place du casque / des inserts en conduction aérienne sur le patient ;
- la sélection des paramètres suivants :
• un ou plusieurs tests à réaliser parmi les tests en stimulation aérienne ou en stimulation vibratoire et, pour chacun desdits tests
• le ou les oreilles à tester,
• la présence ou non d'un masquage controlatéral ;
- sélection du type de transducteur utilisé et de leur positionnement ;
- modification de la calibration des transducteurs selon le transducteur et les paramètres sélectionnés, par application d'une pondération fréquentielle globale des signaux ;
- génération par les transducteurs des signaux fréquentiels, constituant les stimuli de la stimulation vibratoire et/ou aérienne.

2. Procédé de calibration d'un dispositif à transducteurs audiométriques selon la revendication précédente, **caractérisé en ce que** le transducteur vibratoire est placé sur au moins un emplacement de la tête sélectionné parmi les suivants :
- le front ou sur l'axe médian du crâne ;
- le mastoïde ;
- une zone couvrant une portion cartilagineuse de la tête.

3. Procédé de calibration d'un dispositif à transducteurs audiométriques selon l'une des revendications précédentes, **caractérisé en ce que** :
- lorsque la stimulation par transducteur vibratoire a lieu sur le front, la pondération fréquentielle appliquée est comprise entre [-10 et +20] dB ;
- lorsque la stimulation par transducteur vibratoire a lieu sur un mastoïde, la pondération fréquentielle appliquée est comprise entre [-20 et +10] dB ;
- lorsque la stimulation par transducteur vibratoire a lieu sur une zone couvrant une portion cartilagineuse de la tête, la pondération fréquentielle appliquée est comprise entre [-40 et 0] dB.

4. Procédé de calibration d'un dispositif à transducteurs audiométriques selon l'une des revendications 1 à 3, **caractérisé en ce que** la pondération fréquentielle additionnelle appliquée est quasi nulle, c'est-à-dire comprise entre [-5 ; 5] dB si un masquage controlatéral est effectué via l'appareil de stimulation aérienne placé sur l'oreille controlatérale uniquement, l'oreille testée restant libre.

5. Procédé de calibration d'un dispositif à transducteurs audiométriques selon l'une des revendications 1 à 4, **caractérisé en ce que** la pondération fréquentielle appliquée est comprise entre [-30 ; +10] dB si un masquage controlatéral est effectué via l'appareil de stimulation aérienne placé simultanément sur les deux oreilles et que le transducteur vibratoire est positionné sur le front, sur la (les) mastoïde(s) ou sur une zone couvrant une portion cartilagineuse de la tête.

6. Système de fixation à la tête d'un utilisateur de transducteurs pour la réalisation d'une audiométrie en stimulation aérienne par conduction aérienne et en stimulation vibratoire, ladite stimulation vibratoire étant par conduction osseuse ou par conduction dans les tissus cartilagineux mous, ladite audiométrie étant réalisée par un procédé de calibration d'un dispositif à transducteurs audiométriques selon l'une des revendications 1 à 5, **caractérisé en ce qu'**il comporte des moyens support (1, 10, 20, 24) de deux transducteurs audiométriques (21) en stimulation aérienne chacun positionnable au niveau d'un des pavillons auditifs ou du méat acoustique externe et d'au moins un transducteur audiométrique (3, 13, 23) en stimulation osseuse, positionnable(s) au niveau d'au moins une zone de stimulation souhaitée sélectionnée parmi une des mastoïdes du crâne, le front, et une zone couvrant une portion cartilagineuse de la tête.

7. Système de fixation selon la revendication précédente, **caractérisé en ce que** les moyens support comportent une combinaison de premiers moyens support (20) pour les transducteurs audiométriques (21) en stimulation aérienne et de seconds moyens support (1, 10) pour les transducteurs audiométriques en stimulation vibratoire (3, 13).

8. Système de fixation selon la revendication précédente, **caractérisé en ce que** les premiers moyens support (20) des transducteurs audiométriques (21) en stimulation aérienne et les seconds moyens support (24) des transducteurs audiométriques (23) en stimulation vibratoire sont solidarisés l'un à l'autre.

9. Système de fixation selon l'une des revendications 7 à 8, **caractérisé en ce que** les seconds moyens support pour le ou les transducteurs audiométriques (3) en stimulation vibratoire sont constitués d'un bandeau (1) positionnable au-dessus ou au-dessous des pavillons auriculaires.

10. Système de fixation selon l'une des revendications 7 à 8, **caractérisé en ce que** les seconds moyens support pour le ou les transducteurs audiométriques (13) en stimulation vibratoire sont constitués d'un arceau semi-rigide (10) entourant la nuque et dont les extrémités sont munies d'embouts (14) souples placées au-dessus des pavillons auriculaires.

11. Système de fixation selon l'une des revendications 8 à 10, **caractérisé en ce que** les premiers moyens support pour les transducteurs audiométriques (21) en stimulation aérienne consistent en un casque comportant un arceau supérieur (20) muni de moyens réglage de sa longueur et d'écouteurs (21) placés sur les pavillons auriculaires ou dans le conduit auditif externe.

12. Système de fixation selon la revendication précédente, **caractérisé en ce que** l'arceau (20) du casque comporte, soit au voisinage de chaque écouteur (21), soit à sa base, une ou plusieurs branches (24) se développant en direction des zones de stimulation vibratoires (par exemple os mastoïde, front, tissus mous) sur laquelle est solidarisé un transducteur audiométrique (23) en stimulation vibratoire.

13. Système de fixation selon l'une des revendications 6 à 12, **caractérisé en ce que** les transducteurs (21, 3, 13, 23) sont fixés à leurs supports (20, 2,12, 22) via des moyens de fixation dont la position est réglable par rapport auxdits supports (20, 2,12, 22).

## Patentansprüche

1. Verfahren zur Kalibrierung einer am Kopf eines Benutzers angebrachten audiometrischen Wandlervorrichtung zur Luftstimulation und Vibrationsstimulation, zur Durchführung einer Audiometrie durch Aussendung von Signalen mit Frequenzen unter 20 kHz in Richtung des Kopfes;
mit mindestens einem Vibrationswandler von Typ Knochenleitungswandler und einem mit mindestens einem Wandler ausgestatteten Luftstimulationsgerät in einem Kopfhörersatz oder als ein Gehöreinsatz, wobei das Verfahren zur Kalibrierung umfasst:
- Anbringung des oder der Knochenleitungsvibratoren auf dem Kopf des Patienten;
- Anbringung des Kopfhörersatzes / der Luftleitungsgehöreinsätze am Patienten;
- Auswahl der folgenden Parameter:
∘ ein oder mehrere unter den Luftstimulation- oder Vibrationsstimulationstests durchzuführende Tests, für jeden dieser Tests
∘ das oder die zu prüfenden Ohren,
∘ das wohl oder nicht Vorhandensein einer kontralateralen Maskierung;
- Auswahl des verwendeten Wandlertyps und ihrer Positionierung;
- Änderung der Kalibrierung der Wandler je nach ausgewähltem Wandler und den ausgewählten Parametern durch Anwendung einer Gesamtfrequenzgewichtung der Signale;
- Erzeugung durch die Wandler von Frequenzsignalen, die die Anregungsimpulse der Vibrations- und/oder Luftstimulation bilden.

2. Verfahren zur Kalibrierung einer audiometrischen Wandlervorrichtung nach dem vorhergehenden Anspruch,
**dadurch gekennzeichnet, dass** der Vibrationswandler an mindestens einer der folgenden Positionen des Kopfes angebracht ist:
- die Stirn oder auf der Mittellinie des Schädels;
- der Warzenfortsatz;
- ein einen knorpeligen Teil des Kopfes abdeckenden Bereich.

3. Verfahren zur Kalibrierung einer audiometrischen Wandlervorrichtung nach einem der vorhergehenden Ansprüche,
**dadurch gekennzeichnet, dass**:
- wenn die Stimulation durch Vibrationswandler auf der Stirn stattfindet, die angewandte Frequenzgewichtung zwischen [-10 und +20] dB beträgt;
- wenn die Stimulation durch Vibrationswandler auf einem Warzenfortsatz stattfindet, die angewandte Frequenzgewichtung zwischen [-20 bis +10] dB beträgt;
- wenn die Stimulation durch Vibrationswandler an einem einen knorpeligen Teil des Kopfes abdeckenden Bereich stattfindet, die angewandte Frequenzgewichtung zwischen [-40 bis 0] dB beträgt.

4. Verfahren zur Kalibrierung einer audiometrischen Wandlervorrichtung nach einem der Ansprüche 1 bis 3; **dadurch gekennzeichnet, dass** die angewandte zusätzliche Frequenzgewichtung nahezu null ist, d. h. zwischen [-5; 5] dB beträgt, wenn eine kontralaterale Maskierung nur über das Luftstimulationsgerät, das am kontralateralen Ohr angebracht ist, durchgeführt wird, wobei das getestete Ohr frei bleibt.

5. Verfahren zur Kalibrierung einer audiometrischen Wandlervorrichtung nach einem der Ansprüche 1 bis 4; **dadurch gekennzeichnet, dass** die angewandte Frequenzgewichtung zwischen [-30; +10] dB beträgt, wenn eine kontralaterale Maskierung über das Luftstimulationsgerät, das gleichzeitig auf beiden Ohren angebracht wird, durchgeführt wird, und der Vibrationswandler auf der Stirn, auf dem Warzenfortsatz / den Warzenfortsätzen oder auf einem einen knorpeligen Teil des Kopfes abdeckenden Bereich positioniert ist.

6. System zur Befestigung am Kopf eines Benutzers von Wandlern zur Durchführung einer Audiometrie durch Luftstimulation von Luftleitung und durch Vibrationsstimulation, wobei diese Vibrationsstimulation durch Knochenleitung oder durch Leitung im weichen Knorpelgewebe erfolgt; wobei die Audiometrie nach einem Verfahren zur Kalibrierung einer audiometrischen Wandlervorrichtung nach einem der Ansprüche 1 bis 5 durchgeführt wird; **dadurch gekennzeichnet, dass** das System Trägermittel (1, 10, 20, 24) für zwei audiometrischen Wandler (21) in Luftstimulation umfasst, die jeweils an einer der Ohrmuscheln oder des äußeren Gehörgangs positionierbar sind, und mindestens einen audiometrischen Wandler (3, 13, 23) in Knochenstimulation, der, an mindestens einem gewünschten Stimulationsbereich ausgewählt aus einer der Warzenfortsätze des Schädels, der Stirn und einem einen knorpeligen Teil des Kopfes abdeckenden Bereich positionierbar ist.

7. System zur Befestigung nach dem vorhergehenden Anspruch, **dadurch gekennzeichnet, dass** die Trägermittel eine Kombination aus ersten Trägermitteln (20) für die audiometrischen Wandler (21) in der Luftstimulation und zweiten Trägermitteln (1, 10) für die audiometrischen Wandler (3, 13) in der Vibrationsstimulation umfassen.

8. System zur Befestigung nach dem vorhergehenden Anspruch, **dadurch gekennzeichnet, dass** die ersten Trägermittel (20) der audiometrischen Wandler (21) in der Luftstimulation und die zweiten Trägermittel (24) der audiometrischen Wandler (23) in der Vibrationsstimulation miteinander fest verbunden sind.

9. System zur Befestigung nach einem der Ansprüche 7 bis 8, **dadurch gekennzeichnet, dass** die zweiten Trägermittel für den oder die audiometrischen Wandler (3) in der Vibrationsstimulation aus einem Band (1) bestehen, das über oder unter den Ohrmuscheln positionierbar ist.

10. System zur Befestigung nach einem der Ansprüche 7 bis 8, **dadurch gekennzeichnet, dass** die zweiten Trägermittel für den oder die audiometrischen Wandler (13) in Vibrationsstimulation aus einem halbstarren Bügel (10) bestehen, der den Nacken umgibt und dessen Enden mit weichen Stöpseln (14) ausgestattet sind, die über der Ohrmuschel angeordnet sind.

11. System zur Befestigung nach einem der Ansprüche 8 bis 10, **dadurch gekennzeichnet, dass** die ersten Trägermittel für die audiometrischen Wandler (21) in der Luftstimulation einen Kopfhörersatz mit einem Oberbügel (20) mit Längeneinstellmitteln und Kopfhörern (21) umfassen, die auf den Ohrmuscheln oder im äußeren Gehörgang angeordnet sind.

12. System zur Befestigung nach dem vorhergehenden Anspruch, **dadurch gekennzeichnet, dass** der Bügel (20) des Kopfhörersatzes entweder in der Nähe jedes Kopfhörers (21) oder an seiner Basis einen oder mehrere Zweige (24) aufweist, die sich in Richtung der Vibrationsstimulationszonen (z. B. Warzenfortsatz, Stirn, Weichgewebe) ausbreiten, mit der ein audiometrischer Wandler (23) in der Vibrationsstimulation fest verbunden ist.

13. System zur Befestigung nach einem der Ansprüche 6 bis 12, **dadurch gekennzeichnet, dass** die Wandler (21, 3, 13, 23) an ihren Trägern (20, 2,12, 22) über Befestigungsmittel befestigt sind, deren Position in Bezug auf diese Trägern (20, 2,12, 22) einstellbar ist.

## Claims

1. Method for calibrating an audiometric transducer device for air stimulation and vibratory stimulation attached to the user's head for performing audiometry by emitting signals at frequencies below 20 kHz in the direction of the head, comprising at least one vibratory transducer of the bone conduction vibrator type and an air stimulation apparatus provided with at least one transducer, in a pair of headphones or in the form of an auditory insert, the calibration method consisting of :
- Placement of the bone conduction vibrator(s) on the patient's head;
- fitting the air conduction headphone/inserts to the patient;
- selection of the following parameters:
• one or more tests to be carried out using air stimulation or vibratory stimulation and, for each of these tests
• the ear(s) to be tested,
• the presence or absence of contralateral masking;
- selection of the type of transducer used and their positioning;
- modification of the transducer calibration according to the transducer and the parameters selected, by applying a global frequency weighting to the signals;
- generation by the transducers of frequency signals, constituting the stimuli for vibratory and/or aerial stimulation.

2. A method of calibrating an audiometric transducer device according to the preceding claim, **characterized in that** the vibratory transducer is placed on at least one location of the head selected from the following:
- the forehead or on the median axis of the skull;
- the mastoid;
- a zone covering a cartilaginous portion of the head.

3. Method of calibrating an audiometric transducer device according to one of the preceding claims, **characterized in that**:
- when stimulation by vibratory transducer takes place on the forehead, the frequency weighting applied is between [-10 and +20] dB;
- when stimulation by vibratory transducer takes place on a mastoid, the frequency weighting applied is between [-20 and +10] dB;
- when stimulation by vibratory transducer takes place over an area covering a cartilaginous portion of the head, the frequency weighting applied is between [-40 and 0] dB.

4. Method for calibrating an audiometric transducer device according to one of claims 1 to 3, **characterized in that** the additional frequency weighting applied is virtually zero, i.e., between [-5; 5] dB if contralateral masking is performed via the air stimulation device placed on the contralateral ear only, and the test ear is unoccluded.

5. Method for calibrating an audiometric transducer device according to one of claims 1 to 4, **characterized in that** the frequency weighting applied is between [-30; +10] dB if contralateral masking is performed via the air stimulation device placed simultaneously on both ears and the vibratory transducer is positioned on the forehead, on the mastoid(s) or on a zone covering a cartilaginous portion of the head.

6. System for attaching transducers to the head of a user for performing audiometry using air stimulation by air conduction and vibratory stimulation by bone conduction or by conduction in soft cartilaginous tissues, **characterized in that** it comprises support systems (1, 10, 20, 24) for two audiometric transducers (21) for air stimulation, each of which can be positioned at one of the auditory pinnae or the external acoustic meatus, and at least one audiometric transducer (3, 13, 23) for bone stimulation, which can be positioned onto at least one desired stimulation zone selected from one of the mastoids of the skull, the forehead, and a zone covering a cartilaginous portion of the head.

7. Fixing system according to the preceding claim, **characterized in that** the support system comprises a combination of first support system (20) for the audiometric transducers (21) for air stimulation and second support system (1, 10) for the audiometric transducers for vibration stimulation (3, 13).

8. Fixing system according to the preceding claim, **characterized in that** the first support system (20) of the audiometric transducers (21) for air stimulation and the second support system (24) of the audiometric transducers (23) for vibration stimulation are connected to each other.

9. Fixation system according to one of claims 7 to 8, **characterized in that** the second support system for the audiometric transducer or transducers (3) in vibratory stimulation consist of a headband (1) which can be positioned above or below the auricles.

10. Fixation system according to one of claims 7 to 8, **characterized in that** the second support system for the audiometric transducer or transducers (13) in vibratory stimulation consist of a semirigid hoop (10) surrounding the nape of the neck and the ends of which are provided with flexible tips (14) placed above the auricular pinnae.

11. Fixation system according to one of claims 8 to 10, **characterized in that** the first support system for the audiometric transducers (21) for air stimulation consist of a headset comprising an upper headband (20) equipped with means for adjusting its length and earphones (21) placed on the auricular pinnae or in the external auditory canal.

12. A fastening system according to the preceding claim, **characterized in that** the headband (20) comprises, either in the vicinity of each earphone (21), or at its base, one or more branches (24) extending in the direction of the vibratory stimulation zones (for example, mastoid bone, forehead, soft tissues) to which an audiometric transducer (23) is attached for vibratory stimulation.

13. Fixing system according to one of claims 6 to 12, **characterized in that** the transducers (21, 3, 13, 23) are fixed to their support systems (20, 2, 12, 22) via fixing means whose position is adjustable relative to said support systems (20, 2, 12, 22).
